# EUROPEAN PATENT APPLICATION

(11) **EP 3 501 525 A1**
(43) Date of publication of application: **26.06.2019**
(21) Application number: 17841251.6
(22) Date of filing: 13.04.2017
(51) Int. Cl.: A61K 35/28, A61P 9/10, A61P 25/00, A61P 25/28, A61P 43/00

(54) **LIFE EXTENSION AGENT**

(30) Priority: 18.08.2016 JP 2016160269
(71) Applicant: Sapporo Medical University, Sapporo-shi, Hokkaido 060-8556 (JP); Nipro Corporation, Osaka-shi, Osaka 531-8510 (JP)
(72) Inventor: HONMOU, Osamu, Sapporo-shi Hokkaido 060-8556 (JP); SASAKI, Masanori, Sapporo-shi Hokkaido 060-8556 (JP); SASAKI, Yuko, Sapporo-shi Hokkaido 060-8556 (JP); MAEZAWA, Rie, Sapporo-shi Hokkaido 060-8556 (JP); OKA, Shinichi, Sapporo-shi Hokkaido 060-8556 (JP); NAKAZAKI, Masahito, Sapporo-shi Hokkaido 060-8556 (JP)
(74) Representative: Hoffmann Eitle
(86) International application number: PCT/JP2017/015809
(87) International publication number: WO 2018/034023

(57) **Abstract**

The present invention relates to a life extension agent containing CD24-negative mesenchymal stem cells and the treatment of dementia, cerebral infarction, spinal cord injury and the like using the life extension agent.

## Description

### Technical Field

### [Related Applications]

This specification claims priority to Japanese Patent Application No. 2016-160269 (filed on August 18, 2016), the content of which is incorporated herein.

### [Technical Field]

The present invention relates to a life extension agent containing CD24-negative mesenchymal stem cells.

### Background Art

It is known that mesenchymal stem cells (MSCs) have the effect of protecting the brain (parenchyma and blood vessels). It has been confirmed using experimental infarct models that MSC administration after cerebral infarction reduces infarct volume and improves behavioral functions (Non Patent Literatures 1 to 3 and Patent Literature 1). Many treatments of cerebral infarction patients by the intravenous administration of MSCs are also performed, and improvement in motor functions and damaged sites has been reported (Non Patent Literature 4 and Patent Literature 2).

Meanwhile, the promotion of functional recovery and axon regeneration and a reduction in damaged sites by the intravenous administration of MSCs also as to spinal cord-injured patients are observed. Although there have been many reports of effects of MSCs on spinal cord-injured patients in an acute stage until now, patients in a chronic stage have been little investigated, and effects of MSCs on convalescence and lives have not been confirmed sufficiently.

There are many putative action mechanisms as to the therapeutic mechanism of MSCs, and these are classified into three, neurotrophic and neuroprotective effects by neurotrophic factors; vascularization effect (recovery of cerebral blood flow); and neurotization. It is predicted that neurotrophic and neuroprotective effects are exhibited through liquid factors such as BDNF (Brain Derived Neurotrophic Factor) and GDNF (Glial Derived Neurotrophic Factor), which are neurotrophic factors. There are two putative mechanisms of vascularization effect, one is that MSCs accumulating in lesions secrete a vascularization factor or the like and induce vascularization, and the other is that administered MSCs themselves differentiate into vascular endothelium and form new blood vessels. There are also two putative mechanisms of neurotization effect, one is that MSCs accumulating in lesions promote endogenous neurogenesis, and the other is that administered MSCs themselves differentiate into nerve cells and glia cells.

However, all the above-mentioned action mechanisms are just presumption from the observed phenomena, and mechanisms in which cerebral infarction and spinal cord injury are cured by the intravenous administration of MSCs are still unknown.

### Citation List

### Patent Literature

Patent Literature 1:
   International Publication No. WO 2002/000849
Patent Literature 2:
   International Publication No. WO 2009/034708

### Non Patent Literature

Non Patent Literature 1:
   Iihoshi S. et al., Brain Res. 2004; 1007: 1-9.
Non Patent Literature 2:
   Nomura T. et al., Neuroscience. 2005; 136: 161-169.
Non Patent Literature 3:
   Honma T. et al., Exp. Neurol. 2006; 199: 56-66.
Non Patent Literature 4:
   Honmou O. et al., Brain. 2011; 134: 1790-1807.

### Summary of Invention

### Technical Problem

An object of the present invention is to find new effects of mesenchymal stem cells (MSCs) and establish the therapeutic strategies and a new clinical application of MSC transplantation.

### Solution to Problem

The inventors have confirmed that lives are extended by the intravenous administration of MSCs in SHRSPs (stroke-prone spontaneously hypertensive rats). The inventors have confirmed that increase in motor functions by intravenous administration of MSCs also in normal elderly rat is observed. It is presumed from these results that the intravenous administration of MSCs generally has life extension effect regardless of whether subjects have a disease or not.

That is, the present invention relates to the following (1) to (15).
(1) A life extension agent, comprising CD24-negative mesenchymal stem cells derived from bone marrow or blood.
(2) The life extension agent according to the above-mentioned (1), wherein the cells are positive for at least one or more selected from CD73, CD90, CD105 and CD200 and/or negative for at least one or more selected from CD19, CD34, CD45, CD74, CD79α and HLA-DR.
(3) The life extension agent according to the above-mentioned (1) or (2), wherein the cells are derived from bone marrow or blood, preferably human bone marrow or blood.
(4) The life extension agent according to the above-mentioned (3), wherein the bone marrow or the blood is bone marrow or blood from a subject to which the life extension agent is administered.
(5) The life extension agent according to any of the above-mentioned (1) to (4), wherein the cells have been proliferated in a culture medium comprising human serum.
(6) The life extension agent according to the above-mentioned (5), wherein the human serum is autologous serum from the subject to which the life extension agent is administered.
(7) The life extension agent according to any of the above-mentioned (1) to (6), wherein the life extension agent is a preparation for intravenous administration, a preparation for lumbar puncture administration, a preparation for intracerebral administration, a preparation for intraventricular administration, a preparation for local administration or a preparation for intraarterial administration.
(8) The life extension agent according to any of the above-mentioned (1) to (6), wherein the life extension agent is a preparation for intravenous administration.
(9) The life extension agent according to any of the above-mentioned (1) to (8), wherein the cells have been proliferated and enriched in a culture medium containing no anticoagulant or less than 0.02 U/mL of an anticoagulant.
(10) The life extension agent according to any of the above-mentioned (3) to (9), wherein the bone marrow or the blood has been collected by adding an anticoagulant in an amount of less than 0.2 U/mL of the volume of the bone marrow or the blood.
(11) The life extension agent according to the above-mentioned (9) or (10), wherein the anticoagulant is heparin, a heparin derivative or a salt thereof.
(12) The life extension agent according to any of the above-mentioned (1) to (11), wherein the life extension agent improves motor functions and/or cognitive functions of the subject.
(13) The life extension agent according to any of the above-mentioned (1) to (12), wherein the life extension agent increases expression of a FoxO1 gene in brain tissue of the subject.
(14) The life extension agent according to the above-mentioned (13), wherein the life extension agent further increases expression of one or more genes selected from the group consisting of TGF-β1, ALK5 and Smad3.
(15) The life extension agent according to any of the above-mentioned (5), (6) and (9) to (11), wherein the mesenchymal stem cells can secrete TGF-β1 after administration.

### Advantageous Effects of Invention

It has been confirmed that MSCs administered intravenously can improve motor functions and extend lives not only in patients having nerve diseases such as cerebral infarction, spinal cord injury and dementia but also in healthy humans by the present invention. Therefore, MSCs can be administered for the rejuvenation, the life extension (especially of the middle aged and the elderly) and the physical strength improvement (especially of the young) of healthy humans. MSCs can be administered in expectation of life extension effect after the treatment in addition to improvement in nerve functions and motor functions in patients having dementia (vascular dementia and Alzheimer dementia) and intractable neurological disorders such as cerebral infarction and spinal cord injury.

### Brief Description of Drawings

Figure 1 is a Kaplan-Meier curve showing the relationship between the number of days from the administration date when MSCs are administered and the survival rate in SHRSPs. Tests were performed by the log-rank method, and p < 0.05 was defined as a significance level.
Figure 2 shows improvement in the motor functions in elderly rats (17 weeks old) by administering MSCs (-●-: MSC-treated group, -■-: DMEM-treated group).
Figure 3 shows improvement in the survival rate in elderly rats (50 weeks old) by administering MSCs (-●-: MSC- treated group, -■-: DMEM- treated group).
Figure 4 shows improvement in the motor functions in elderly (50 weeks old) rats by administering MSCs (-●-: MSC- treated group, -■-: DMEM- treated group) .
Figure 5 shows improvement in the cognitive functions in elderly rats (50 weeks old), 10 weeks after administration of MSCs (-●-: MSC- treated group, -■-: DMEM- treated group).
Figure 6 shows the results of immunostaining of rat brain tissue after administration of MSCs. (A) DAPI/GFP, (B) DAPI/TGF-β1, (C) Merge (GFP/TGF-β), (D) DAPI/GFP (MSC/TGF-β1
Figure 7 shows the result of the immunostaining of rat brain tissue after administration of MSCs. GFP (MSC/TGF-β1
Figure 8 shows changes in the expression of genes by administration of MSCs in SD rats. (A) FoxO1 (longevity gene), (B) TGF-β1, (C) ALK5, (D) Smad5.
Figure 9 shows a mechanism in which MSCs improve a life (presumption).

### Description of Embodiments

### [Mesenchymal stem cell]

It is known that the "mesenchymal stem cells" used in the present invention are stem cells existing in the interstitial cells of mesenchymal tissue in a very small amount and having multipotential and self-replicating ability, and not only differentiate into connective tissue cells such as bone cells, cartilage cells, and fat cells, but also have the ability to differentiate into nerve cells or cardiac muscle cells.

Although the mesenchymal stem cells exist throughout the body in bone marrow, peripheral blood, umbilical cord blood, the fetus, the placenta, fat, the brain, etc. mesenchymal stem cells derived from bone marrow or blood (bone marrow mesenchymal stem cells), especially mesenchymal stem cells derived from human bone marrow or human blood (human bone marrow mesenchymal stem cells), are preferable in the present invention. There are advantages 1) the bone marrow mesenchymal stem cells can be expected to have a remarkable effect, 2) the bone marrow mesenchymal stem cells have a low risk of side effects, 3) enough donor cells can be expected to be supplied, 4) the bone marrow mesenchymal stem cells allow noninvasive treatments and autotransplantation, 5) the bone marrow mesenchymal stem cells have a low risk of infection, 6) the bone marrow mesenchymal stem cells may not cause immunological rejection, 7) the bone marrow mesenchymal stem cells have no ethical problems, 8) the bone marrow mesenchymal stem cells are easily accepted socially, 9) the bone marrow mesenchymal stem cells are easily established as a general medical service, etc. Further, a bone marrow transplantation is a treatment which has already been used at clinical sites, and the safety thereof has been also confirmed. The bone marrow-derived stem cells are highly migratory, reach damaged tissue to be targeted, not only by transplantation to a part but also by intravenous administration, and can be expected to have therapeutic effects.

The cells may be cells differentiation-induced from ES cells or induced pluripotent stem cells (iPS cells or the like), cells from an established cell line, or cells isolated and proliferated from the living body. The cells can be collected from any tissue in the living body (such as fat, the umbilical cord, the placenta, the amnion or the like), and are not particularly limited. Although the cells may be allogeneic or autologous, autologous (a patient's own cell-derived) mesenchymal stem cells are preferable.

The mesenchymal stem cells used in the present invention are negative for CD24, which is a differentiation marker, and are cells remaining undifferentiated. Therefore, the mesenchymal stem cells have the characteristics of a high reproduction rate and a high survival rate after introduction into the living body. The inventors have also developed a method for obtaining such undifferentiated mesenchymal stem cells, and the details thereof are described in International Publication No. WO 2009/002503.

Mesenchymal stem cells used in the present invention are characterized by being positive for at least one or more selected from CD73, CD90, CD105 and CD200 and/or negative for at least one or more selected from CD19, CD34, CD45, CD74, CD79α and HLA-DR besides CD24. Preferably, mesenchymal stem cells used in the present invention are characterized by being positive for two or more of CD73, CD90, CD105 and CD200 and negative for four or more of CD19, CD34, CD45, CD74, CD79α and HLA-DR. More preferably, mesenchymal stem cells used in the present invention are characterized by being positive for CD73, CD90, CD105 and CD200 and negative for CD19, CD34, CD45, CD74, CD79α and HLA-DR.

In the method which the inventors have developed, cells separated from bone marrow liquid or the like under the conditions that the cells do not substantially contact with an anticoagulant (heparin or the like) are proliferated using a culture medium containing human serum (preferably autologous serum) and containing no anticoagulant (heparin or the like) or an anticoagulant at an extremely low concentration.

The density of cells in the culture medium affects the properties of the cells and the direction of differentiation. In the case of mesenchymal stem cells, when the cell density in a culture medium exceeds 8,500 cells/cm², the properties of the cells change. Therefore, it is preferable to subculture the cells at 8,500 cells/cm² or less at the maximum. It is more preferable to subculture the cells at the time of 5,500 cells/cm² or more.

In the method which the inventors have developed, since a culture medium containing human serum is used, it is desirable that the culture medium be exchanged as few times as possible in light of the burden for a serum donor, and the culture medium is replaced, for example, at least once a week, more preferably once or twice a week.

Subculture is repeated until the total number of the cells reaches 10⁸ cells or more. Although the number of the required cells depends on the intended use, the number of the mesenchymal stem cells required for transplantation for the treatment of cerebral infarction is believed to be, for example, 10⁷ cells or more. According to the method which the inventors have developed, 10⁷ mesenchymal stem cells can be obtained in about 12 days.

Proliferated MSCs may be stored by a technique such as cryopreservation (for example, in a deep freezer at - 152°C) until use if needed. A culture medium containing serum (preferably human serum and more preferably autologous serum), dextran and DMSO (culture medium for mammalian cells, such as a RPMI medium) is used as cryopreservation solution. For example, the cells can be suspended in a cryopreservation solution containing 20.5 mL of a usual filtration-sterilized RPMI medium, 20.5 mL of autologous serum collected from a patient, 5 mL of dextran and 5 mL of DMSO and cryopreserved at -150°C. For example, Cryoserv produced by NIPRO CORPORATION is available for the DMSO, and a Low Molecular Dextran L produced by Otsuka Pharmaceutical Co., Ltd. is available for the dextran, but the DMSO and the dextran are not limited to these products.

MSCs of the present invention can secrete TGF-β1 after administration. It is presumed that the administration of MSCs increase the motor functions, the cognitive function and the survival time of the subject by activating a TGF-β1/Smad3 pathway in target tissue (for example, brain tissue) and increasing the expression of a FoxO1 gene, which is called a longevity gene.

### [Life extension agent]

The "life extension agent" of the present invention is a cell preparation containing mesenchymal stem cells (MSCs) negative for CD24, and is a medicine which has the effect of extending the life of the administered subject. As mentioned below, the "life extension agent" of the present invention has life extension effect through various effects such as improvement in motor functions in healthy humans and has life extension effect after the treatment in addition to improvement in nerve functions and motor functions in patients having dementia and intractable neurological disorder such as cerebral infarction and spinal cord injury.

The higher number of the MSCs contained in the life extension agent of the present invention is preferable. However, the life extension agent containing the minimum number which exhibits intended effect is practical considering time of administration to a subject and time required for culture. Therefore, in a preferred aspect of the life extension agent of the present invention, the number of mesenchymal stem cells is 10⁷ cells or more, preferably 5 × 10⁷ cells or more, more preferably 10⁸ cells or more, and further preferably 5 × 10⁸ cells or more.

The life extension agent of the present invention is preferably a preparation for parenteral administration and more preferably a preparation for parenteral systemic administration, especially a preparation for intravenous administration. Examples of dosage forms suitable for parenteral administration include injections such as a solution injection, a suspension injection, an emulsion injection and an injection prepared before use; and transplants. The preparation for parenteral administration is in the form of a aqueous or nonaqueous isotonic aseptic solution or a suspension, and is formulated in a proper unit dosage form appropriately in combination of, for example, pharmacologically acceptable carriers or media, specifically sterile water and physiological saline; a culture medium (culture medium such as especially a RPMI medium used for culturing mammalian cells); physiological buffer solution such as PBS; a vegetable oil; an emulsifier; a suspending agent; a surfactant; a stabilizer; an excipient; a vehicle; an antiseptic; a binding material; and the like.

Examples of the solution for injection include physiological saline; a culture medium; physiology buffer solution such as PBS; isotonic solution containing glucose and other adjuvants, and examples include D-sorbitol, D-mannose, D-mannitol and sodium chloride. The solution for injection may be used in combination with a proper solubilizer, for example, alcohol, specifically ethanol, polyalcohol, propylene glycol or polyethylene glycol; and a nonionic surfactant such as polysorbate 80 and HCO-50.

The life extension agent of the present invention can improve motor functions and simple cognitive functions, improve higher order function such as disturbance of attention, memory disorder, aphasia, forgetfulness, apraxia, disturbance in executive functioning, and emotional disorder, and extend the lives of subjects after medical treatments in the patients having dementia, cerebral infarction, spinal cord injury, neurodegenerative disease and mental disorder. Further, the life extension agent also has the effect of rejuvenation, physical strength improvement, and life extension in healthy humans.

Dementia is a disorder wherein normally developed intelligence decreases irreversibly due to acquired brain injury, and the patients exhibit cognitive disorder. Examples of dementia include Alzheimer dementia, vascular dementia, Lewy body dementia, and frontotemporal dementia. Since the life extension agent of the present invention can improve the general quality of life in addition to improvement in a cognitive function (Japanese Patent Application No. 2016-091286 and Japanese Patent Application No. 2016-091300) in dementia patients, the remaining lives of the dementia patients can be expended.

Cerebral infarction means a pathosis wherein the occlusion or the stenosis of a brain artery causes cerebral ischemia, and brain tissue becomes necrosed or almost necrosed. MSCs have the effect of protecting the brain (parenchyma and blood vessels), and the intravenous administration of MSC reduces infarct volume and improves a behavioral function in cerebral infarction in an acute stage or a subacute stage. Necrosed cells and injured nerve fibers are irreversible when cerebral infarction reaches a chronic stage. Therefore, it has been believed that it is important for medical treatments to prevent the recurrence, restore live cells and cells in abeyance which exist around the necrosed cells, and alleviate the condition of a disease in cerebral infarction in a chronic stage. However, the intravenous administration of MSCs can extend the remaining lives of subjects in addition to the restoration of motor functions and cerebral functions (Japanese Patent Application No. 2016-091286 and Japanese Patent Application No. 2016-091300) also in cerebral infarction in a chronic stage by promoting the reconstruction of neural circuits and the compensation of normal tissue. When patients become bedridden, general prostration advances, resulting in the hypofunction of not only muscles but also internal organs, but it is because MSC treatment enables locomotion which can in turn recover and maintain the health of the whole body including muscles and internal organs.

The central nervous system including the spinal cord is not restored or regenerated once the central nervous system is injured, which is different from the case of peripheral nerves. The treatment of especially spinal cord injury in a chronic stage with advanced scarring is difficult. Although clinical tests using ES cells have also been attempted, the tests have not succeeded yet. However, the intravenous administration of MSCs can extend the remaining lives of subjects in addition to the restoration of motor functions and nerve functions also in spinal cord injury in a chronic stage (Japanese Patent Application No. 2016-091286 and Japanese Patent Application No. 2016-091300) by promoting the reconstruction of neural circuits and the compensation by normal tissue. As mentioned above, when patients become bedridden, general prostration advances, resulting in the hypofunction of not only muscles but also internal organs. However, MSC treatment enables locomotion, which can in turn recover and maintain the health of the whole body including muscles and internal organs.

The life extension agent of the present invention is also useful for neurodegenerative disorders such as amyotrophic lateral sclerosis (ALS), Parkinson's disease, progressive supranuclear palsy (PSP), Huntington's disease, multiple system atrophy (MSA), striatonigral degeneration (SND), a Shy-Drager syndrome, olivopontocerebellar atrophy (OPCA) and spinocerebellar degeneration (SCD).

The life extension agent of the present invention is also useful for mental disorders such as schizophrenia, manic-depressive insanity, personality disorder, mood disorder, mental developmental disorder, stress-related disorder, autism, learning disability, behavior and emotional disorder, mental retardation, sleep disorder, eating disorder, identity disorder, dissociative disorder, maladjustment, alcohol disorder, and alcohol dependence.

### Examples

Although the present invention will be described in detail hereinafter by Examples, the present invention is not limited to these examples.

### Example 1. Life extension effect in stroke-prone spontaneously hypertensive rats (SHRSPs)

### 1. Materials and Methods

### (1) Preparation of mesenchymal stem cells derived from rat bone marrow

The experiment was performed according to the animal experiment management rules of Sapporo Medical University. According to the previous report, bone marrow obtained from the femurs of mature SD rats was diluted with Dulbecco's modified Eagle's medium (DMEM) to 25 ml, and 10% heated and inactivated FBS, 2 mM 1-glutamine, 100 U/ml penicillin and 0.1 mg/ml streptomycin were added. The cells were incubated in a 5% CO₂ atmosphere at 37°C for 3 days (Kim S. et al., Brain Res. 2006; 1123:27-33. Ukai R. et al., J. Neurotrauma. 2007; 24:508-520.). The cells were cultured until the cells became confluent, and adherent cells were detached with trypsin-EDTA and subcultured at a density of 1 × 10⁴ cells/ml 3 times to obtain mesenchymal stem cells (MSCs).

### (2) Stroke-prone spontaneously hypertensive rat (SHRSP)

SHRSPs (Stroke-prone spontaneously hypertensive rats) were purchased from Hoshino Laboratory Animals, Inc. These rats are stroke-prone spontaneously hypertensive rats established by selecting and crossbreeding mouse pups of parents the ancestors of which died of a stroke from generation to generation, suffer from the breakdown of the blood brain barrier due to hypertension, develop cerebral infarction, cerebral hemorrhage or dementia, and are short-lived.

### (3) Evaluation of survival rate

Rats which had developed cerebral infarction or cerebral hemorrhage at the time of 16 to 20 weeks were selected as subjects and divided into an MSC group and a DMEM group.
MSC group (culture medium + MSC; n = 8):
   MSCs (1.0 × 10⁶ cells) diluted with a fresh culture medium (DMEM) to a total volume of 1 ml were administered intravenously.
DMEM group (only culture medium; n = 8):
   A fresh culture medium (DMEM; 1 ml) was administered intravenously.

Cyclosporin A (10 mg/kg) was intraperitoneally administered to all the rats every day. The rats were all intravenously administered from the left femoral vein. The numbers of days from MSC or DMEM administration dates to death dates were evaluated in the Kaplan-Meier method. Tests were performed by the log-rank method, and p < 0.05 was defined as a significance level.

### 2. Result

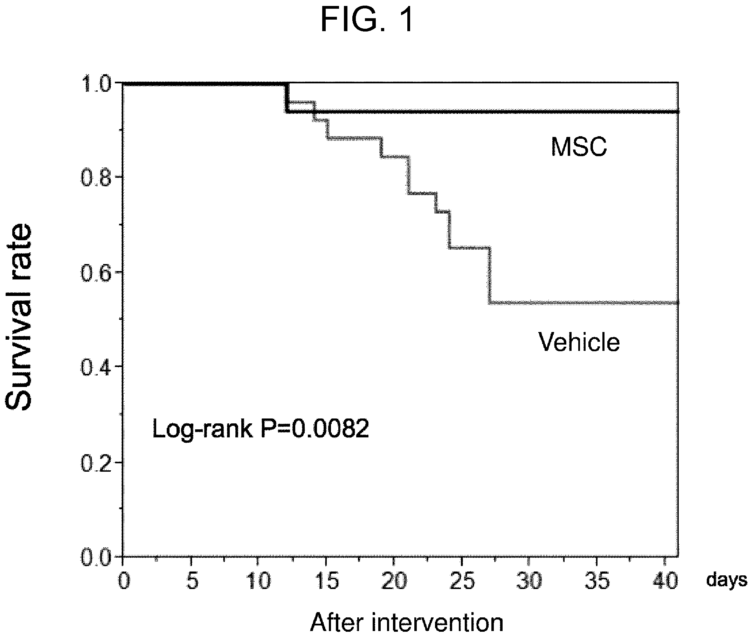

As shown in Figure 1, it was confirmed that lives were extended more remarkably in the MSC group than those in the DMEM group (Log-rank method p = 0.0082). From this result, it was confirmed that MSC administration enables not only the recovery of nerve functions and motor functions but also the extension of lives in a cerebral infarction model.

### Example 2. Increase in motor functions in elderly rats

Although the blood vessels of SHRSPs age spontaneously by feeding high salt diet, blood pressure becomes high, and the SHRSPs finally come to develop cerebral infarction, cerebral hemorrhage and dementia, this also resembles the phenomena of aging of humans. Therefore, the life extension of the SHRSPs by the intravenous administration of MSCs suggests that the MSCs have the life extension effect also in the normal elderly. Accordingly, the effect of MSC on motor functions was evaluated using elderly rats (17 weeks old) in this example.

### 1. Materials and Methods

MSCs were prepared according to Example 1. Cerebral infarction was induced in the method described in Example 3 when the rats were 9 weeks old, and the SD rats (17 weeks old) 8 weeks after that were divided into two groups at random as follows.
MSC group (physiological saline + MSC; n = 8):
   MSCs (1.0 × 10⁶ cells) diluted with the culture medium (DMEM) to a total volume of 1 ml were administered to the 17-week old SD rats from the left femoral vein.
DMEM group (culture-medium; n = 8):
   Fresh culture solution (DMEM; 1 ml) was administered to the 17-week old SD rats from the left femoral vein.

The rats were not subjected to a physical therapy, but cyclosporin A (10 mg/kg) was administered every day for one week after transplantation and administered every other day thereafter. Exercise evaluation was performed on a treadmill (angle 20°) every week.

### 2. Result

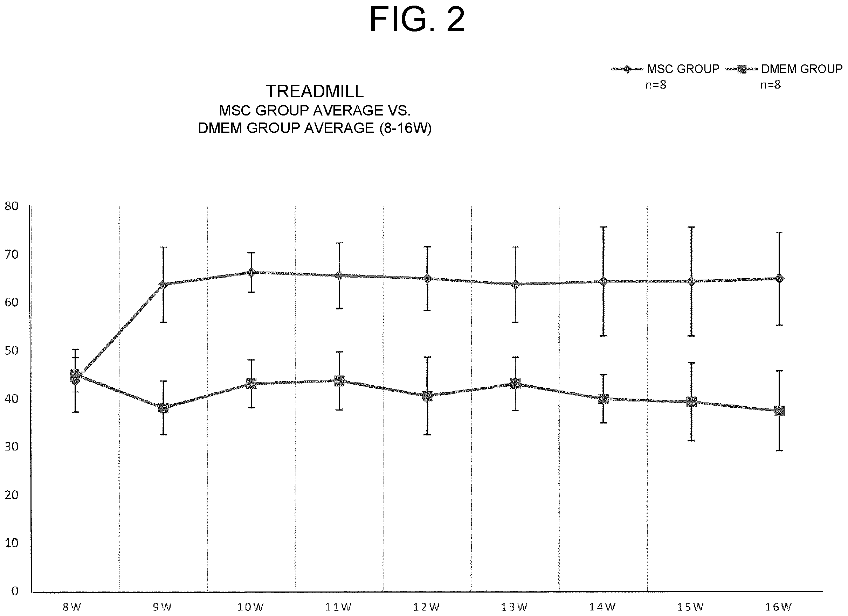

As shown in Figure 2, the motor functions of rats having good motor functions originally were maintained, and the motor functions of rats having low motor functions were improved in the MSC group. Meanwhile, it was observed that the motor functions decreased gradually in the DMEM group. Therefore, it can be expected that MSC administration suppresses decrease in motor functions due to aging, and extends lives also in healthy humans.

### Example 3. Therapeutic effect in cerebral infarction model rats

A rat transient middle cerebral artery occlusion (tMCAO) model was used as a cerebral infarction model. According to the previous report, a mature female SD rat (200 to 250 g) was anesthetized with ketamine (75 mg/kg) and xylazine (10 mg/kg), and 20.0 to 22.0-mm embolism thread (MONOSOF) was inserted from the external carotid artery, resulting in the induction of transient middle cerebral artery occlusion (Honma T. et al., Exp. Neurol. 2006; 199:56-66. Sasaki M. et al., Methods Mol. Biol. 2009; 549:187-195.). The life extension effect of MSC administration on cerebral infarction can be produced according to Example 1 using this rat as a cerebral infarction model.

### Example 4. Therapeutic effect in chronic stage spinal cord injury model

According to the previous report, a chronic stage spinal cord injury model rat can be generated by anesthetizing a mature male SD rat with ketamine (90 mg/kg) and xylazine (4 mg/kg), exposing the spinal cord at a T9 to T10 level by laminectomy, and pressuring and crushing the spinal cord using a spinal cord impactor (Infinite Horizon Impactor, 60-kilodyne) (Matsushita et al., 2015). The life extension effect of MSC administration on dementia can be produced by this rat chronic stage spinal cord injury model according to Example 1.

### Example 5. Life extension effect in elderly rats

The effect of MSC administration on the survival rate, motor functions and cognitive functions was evaluated using 50-week old normal rats.

### 1. Materials and Methods

MSCs were prepared according to Example 1. SD rats which were not subjected to any operation were divided into two groups at random as follows, and MSCs or the culture medium (DMEM) was administered.
MSC group (physiological saline + MSC; n = 15):
   MSCs (1.0 × 10⁶ cells) diluted with the culture medium (DMEM) to a total volume of 1 ml were administered to the 50-week old SD rats from the left femoral vein.
DMEM group (culture medium; n = 15):
   Fresh culture solution (DMEM; 1 ml) was administered to the 50-week old SD rats from the left femoral vein.

All the rats were not subjected to a physical therapy, but cyclosporin A (10 mg/kg) was intraperitoneally administered every day for one week from MSC administration and intraperitoneally administered every other day thereafter.

### (1) Evaluation of survival rate

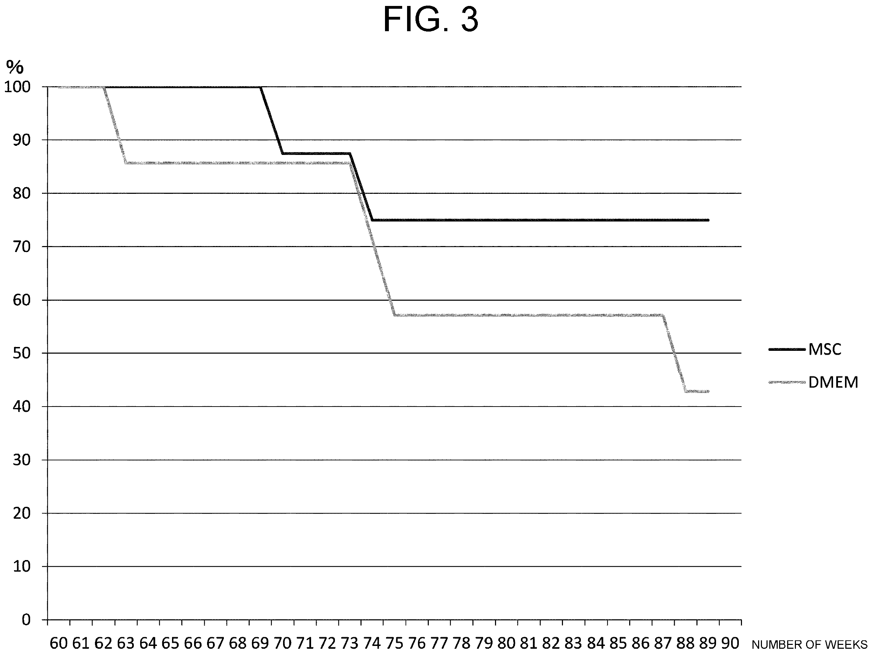

The numbers of days from MSC (n = 10) or DMEM (n = 9) administration dates to death dates were evaluated in the Kaplan-Meier method. The results are shown in Figure 3.

### (2) Evaluation of motor functions

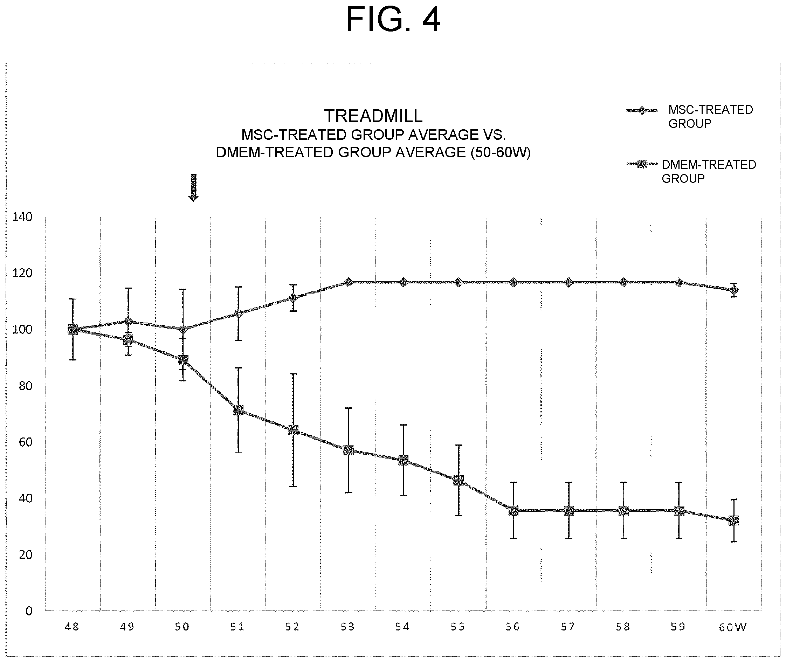

According to Example 2, the MSC group (n= 10) and the DMEM group (n= 9) were subjected to exercise evaluation on a treadmill (angle 20°) every week. The results are shown in Figure 4.

### (3) Evaluation of cognitive functions

The effect of MSC administration on the cognitive functions of elderly rats was verified by the water maze examination (Morris Water Maze Test (MWM) test). A circular pool having a diameter of 1.3 m was filled with cloudy opaque water at a water temperature of 24°C so that the depth was 30 cm. A platform used as a goal was placed directly under the water surface. A rat was placed in from the end of the pool, and time until the rat reaches the goal was measured.

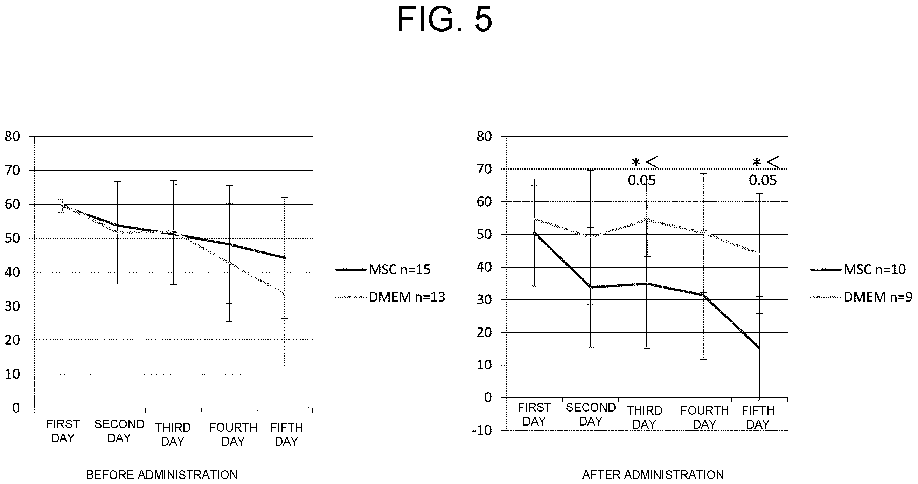

Measurement was performed by video tracking (Anymaze tracking software (Stoelting Co.) for 5 days before the transplantation and continuously for 6 days from the tenth week after the transplantation. Device acclimatization at 4 cycles a minute was performed on the first day, and arrival time was measured continuously for 5 days from the second day. Measurement at 4 cycles a day was performed, and the average value was defined as a measured value. The results are shown in Figure 5.

### 2. Result

As shown in Figures 3 to 5, remarkable improvement in survival rates, motor functions, and cognitive functions was confirmed in the MSC group in comparison with those in the DMEM group. It was confirmed from this result that MSC administration has life extension effect also in elderly normal rats.

### Example 6. Change in gene expression by MSC administration

### 1. Materials and Methods

Rats which had developed cerebral infarction or cerebral hemorrhage at the time of 16 to 20 weeks among the dementia model animals (SHRSPs) used in Example 1 were used as subjects and divided into an MSC group and a DMEM group 2 weeks after crises. Then, 1.0 × 10⁶ cells/ml·DMEM of GFP-labeled MSCs (MSC-GFP) were administered from the left femoral vein. The rats were sacrificed one week after MSC-GFP administration and perfused with 200 ml of PBS, followed by the extraction of brain tissue.

The extracted brain tissue was frozen in isopentane (shock frozen) and preserved at -80°C before use. The brain tissue was sliced to 30 µm, and coronal specimens were prepared and immunostained. As primary antibodies, an anti-GFP antibody (chicken polyclonal antibody (1:1000; Abcam, ab13970)) and an anti-TGF-β1 antibody (rabbit polyclonal antibody (1:100; Abcam, ab92486)) were used, and as secondary antibodies, AF488 conjugate goat anti-chicken immunoglobulin (1:1000) and AF594 conjugate goat anti-rabbit immunoglobulin (1:1000) were used for immunostaining.

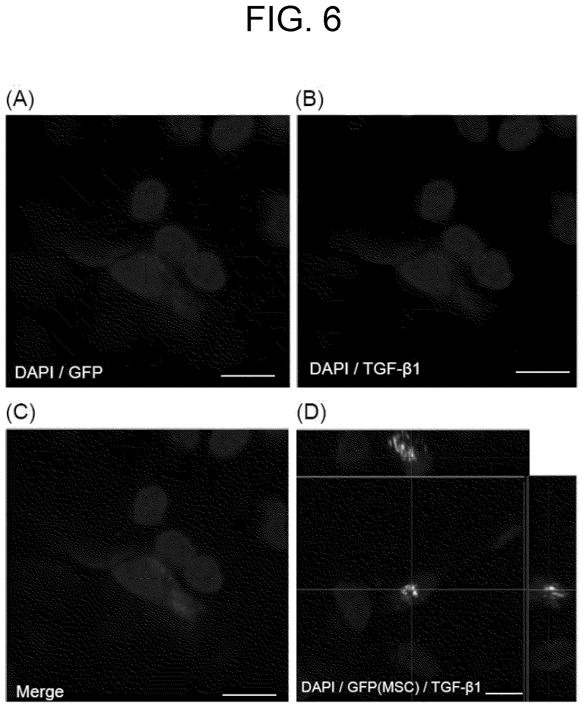

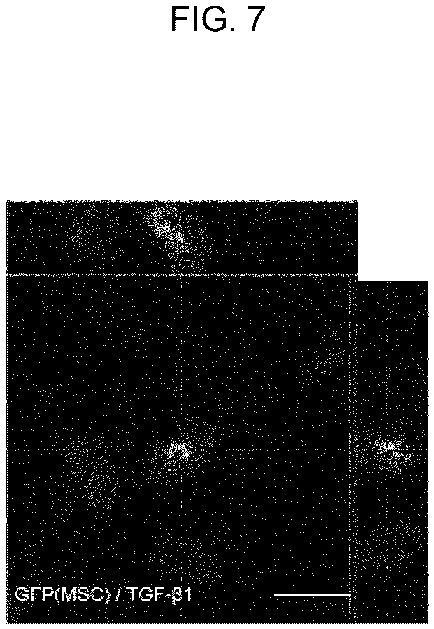

The specimens after immunostaining were observed through an LSM780 confocal laser scanning microscope (Laser: Argon 488 (GFP), 561 (TGF-β1), Objective: Plan-Apochromat 10 ×/0.45 M27, Zeiss). The results are shown in Figures 6 and 7.

### 2. Result

GFP and TGF-β1 were coexpressed in the brain tissue after MSC administration. This result suggests that TGF-β1 is secreted from MSCs.

### Example 7. Change in gene expression by MSC administration

### 1. Materials and Methods

Samples were prepared according to Example 6. Rats were divided into two groups at random as follows, and MSCs or a culture medium (DMEM) was administered to the rats.
MSC group (physiological saline + MSC; n = 4):
   MSCs (1.0 × 10⁶ cells) diluted with the culture medium (DMEM) to a total volume of 1 ml were administered to the rats from the left femoral vein.
DMEM group (culture medium; n = 4):
   Fresh culture solution (DMEM; 1 ml) was administered to the rats from the left femoral vein.

All the rats were not subjected to a physical therapy, but cyclosporin A (10 mg/kg) was intraperitoneally administered every day for 1 week from MSC administration.

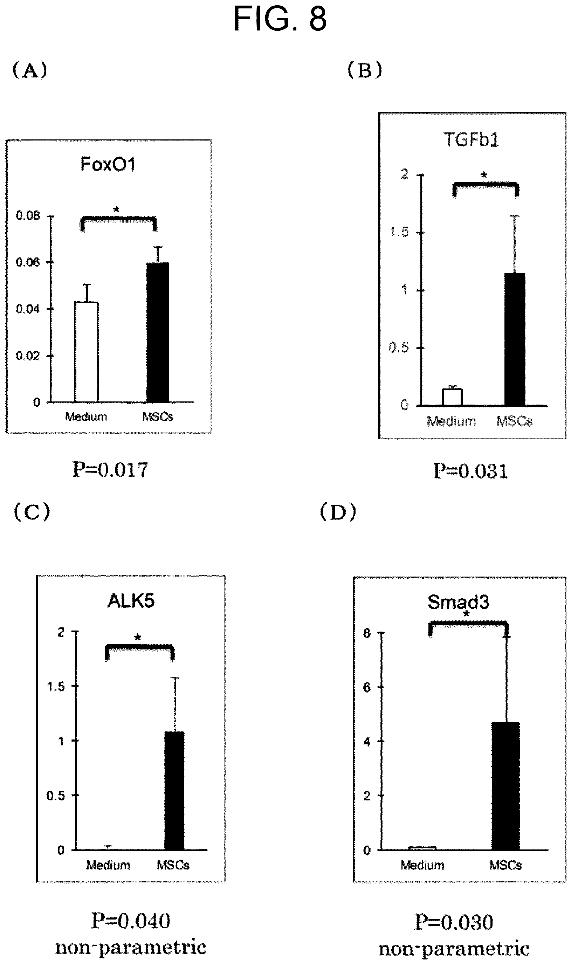

The rats were sacrificed one week after administration, and 20 to 30 mg of cortexes were collected rapidly, and RNA was extracted using an RNeasy Plus Mini kit (Qiagen Corporation). Subsequently, cDNA was prepared from 1 µg of RNA using a SuperScript III reverse transcriptase (Qiagen Corporation). A TaqMan probe (Thermo Fisher Scientific Inc.) was purchased for primers, and RT-PCR was performed using a TaqMan Universal Master Mix II with UNG. The expression of FoxO1, TGF-β1, ALK5, and Smad3 was analyzed using GAPDH (TaqMan rodent GAPDH control reagents) as an endogenous control. PCR was performed using an ABI-StepOne Real-Time PCR system (Thermo Fisher Scientific Inc.). A cycle was heating at 95°C for 3 minutes, then at 95°C for 15 seconds, and at 55°C for 60 seconds. This cycle was repeated 40 times. The amounts of genes expressed were evaluated by the Ct comparative method. The results are shown in Figure 8.

### 2. Result

The MSC group expressed all of FoxO1, TGF-β1, ALK5 and Smad3 significantly more highly than the DMEM group (Figure 8, the following table 1).

**[Table 1]**

| | MSC group | DMEM group | p value |
|---|---|---|---|
| Fox01 | 0.060±0.012 | 0.043±0,015 | P=0.02 |
| TGF-*β*1 | 1.141±0.998 | 0.136±0,056 | P=0.03 |
| ALK5 | 1.086±1.269 | 0.015±0,009 | P=0.04 |
| Smad3 | 4.682±6.375 | 0.114±0.0668 | P=0.03 |

### 3. Consideration

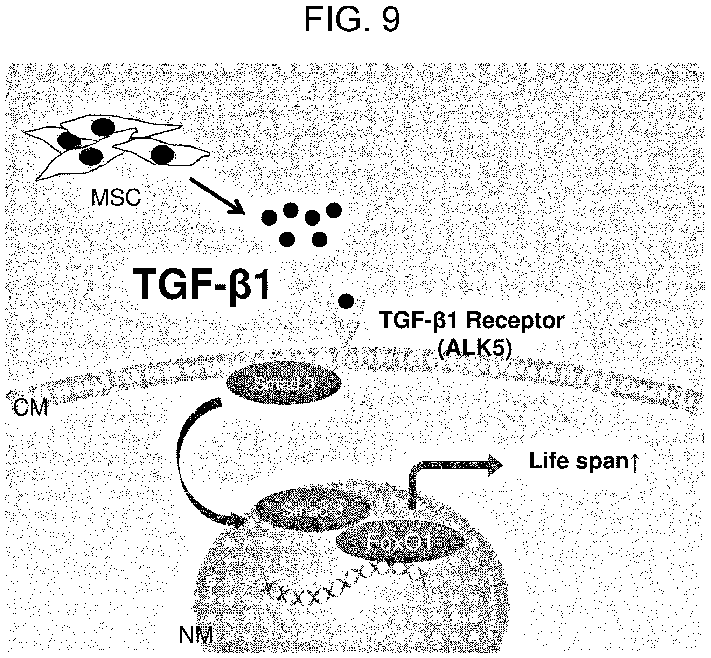

It has been suggested that the TGF-β1/Smad3 pathway may be activated, and the expression of FoxO1 may be increased by the administration of MSCs (Figure 9). This result suggests that MSCs may extend the survival periods of subjects by activating FoxO1, which has gained attention as a longevity gene.

### Industrial Applicability

The present invention is useful for the treatment of subjects desired to live longer. Since the lives of subjects can be extend in addition to improvement in nerve functions and motor functions especially in neurodegenerative diseases such as dementia, cerebral infarction, and spinal cord injury; and mental disorders, the present invention enables establishment of new therapeutic strategies using MSCs. Additionally, in healthy subjects, the motor functions of the subjects can be improved, the lives of the subjects can be extended, and therefore, the administration of MSCs is useful to extend the lives of the subjects regardless of whether the subjects have a disease or not.

All the publication, the patents and the patent applications cited herein are incorporated herein by reference.

## Claims

1. A life extension agent, comprising CD24-negative mesenchymal stem cells.

2. The life extension agent according to claim 1, wherein the cells are positive for at least one or more selected from CD73, CD90, CD105 and CD200 and/or negative for at least one or more selected from CD19, CD34, CD45, CD74, CD79α and HLA-DR.

3. The life extension agent according to claim 1 or 2, wherein the cells are derived from bone marrow or blood.

4. The life extension agent according to claim 3, wherein the bone marrow or the blood is bone marrow or blood from a subject to which the life extension agent is administered.

5. The life extension agent according to any one of claims 1 to 4, wherein the cells have been proliferated in a culture medium comprising human serum.

6. The life extension agent according to claim 5, wherein the human serum is autologous serum from the subject to which the life extension agent is administered.

7. The life extension agent according to any one of claims 1 to 6, wherein the life extension agent is a preparation for intravenous administration, a preparation for lumbar puncture administration, a preparation for intracerebral administration, a preparation for intraventricular administration, a preparation for local administration or a preparation for intraarterial administration.

8. The life extension agent according to any one of claims 1 to 6, wherein the life extension agent is a preparation for intravenous administration.

9. The life extension agent according to any one of claims 1 to 8, wherein the cells have been proliferated and enriched in a culture medium containing no anticoagulant or less than 0.02 U/mL of an anticoagulant.

10. The life extension agent according to any one of claims 3 to 9, wherein the bone marrow or the blood has been collected by adding an anticoagulant in an amount of less than 0.2 U/mL of the volume of the bone marrow or the blood.

11. The life extension agent according to claim 9 or 10, wherein the anticoagulant is heparin, a heparin derivative or a salt thereof.
